# EUROPEAN PATENT APPLICATION

(11) **EP 4 130 732 A1**
(43) Date of publication of application: **08.02.2023**
(21) Application number: 21780596.9
(22) Date of filing: 01.04.2021
(51) Int. Cl.: G01N 30/02, G01N 30/89, C07K 16/46, C12N 15/13, C12P 21/08

(54) **ANALYSIS METHOD FOR IMPURITY MOLECULES IN COMPOSITION CONTAINING MULTI-SPECIFIC ANTIGEN-BINDING MOLECULES**

(30) Priority: 02.04.2020 JP 2020066824
(71) Applicant: CHUGAI SEIYAKU KABUSHIKI KAISHA, Tokyo 115-8543 (JP)
(72) Inventor: SATO, Motohiko, Gotemba-shi, Shizuoka 412-8513 (JP); MURATA, Hiroko, Gotemba-shi, Shizuoka 412-8513 (JP)
(74) Representative: Vossius & Partner Patentanwälte Rechtsanwälte mbB
(86) International application number: PCT/JP2021/014120
(87) International publication number: WO 2021/201202

(57) **Abstract**

The present disclosure provides an analysis method for measuring the content of light chain-exchanged molecules in a composition containing a multi-specific antigen-binding molecule. The analysis method of the present disclosure includes the steps of treating a composition comprising a multi-specific antigen-binding molecule and preparing a plurality of types of F(ab) fragments; and measuring the F(ab) fragments by a separation method based on electric charge or hydrophobic interactions and determining the content (content ratio) of each fragment.

## Description

### [Technical Field]

The present disclosure relates to a method for analyzing impurity molecules in a composition comprising a multi-specific antigen-binding molecule. More specifically, the present disclosure relates to a method for quantifying the content of a light chain-exchanged molecule generated due to light-chain mispairings in a multi-specific antigen-binding molecule in a composition comprising the multi-specific antigen-binding molecule, the method comprising the steps of treating the molecules comprised in the composition and generating multiple F(ab) fragments; and measuring the F(ab) fragments and determining the content or content ratio of each of the fragments.

### [Background Art]

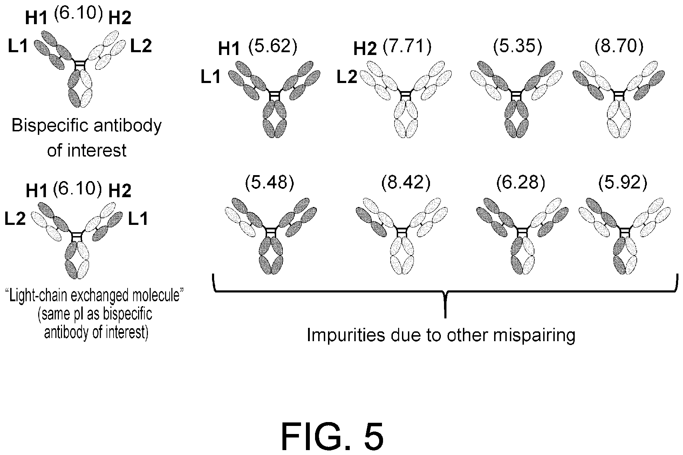

In the case of a multi-specific antigen-binding molecule with non-common light chains (particularly a bispecific antibody), which has two different heavy chains (H1 and H2) and two different light chains (L1 and L2), nine types of impurity molecules are generated during the culturing process, in addition to the molecule of interest (H1L1/H2L2), because of mispairing between the heavy chains or between the heavy and light chains (Fig. 5). To decrease these impurity molecules, the technique referred to as Knobs-into-holes (PTL 1) and the techniques utilizing charge control between heavy chains or between heavy and light chains (PTL 2 and PTL 3) are known. Also, there is a known technique of introducing modifications into amino acid residues of the heavy and/or light chains and removing generated impurity molecules by chromatographic separation using a pI difference between the molecule of interest and the impurity molecules (PTL 4).

However, although the separation method using pI differences allows for separation of eight types of the impurity molecules, the remaining one type in which L1 and L2 that should be paired with H1 and H2 are inverted (H1L2/H2L1: referred to as "light chain-exchanged molecule" hereinafter) cannot be separated because it has the same physical properties as the molecule of interest, including theoretical pI. Thus, from a quality control standpoint, there is a demand for a method for accurately determining the presence or absence and the content or content ratio of light chain-exchanged molecules contained in a composition obtained after purification process.

Yin *et al.* discloses a method for analysis of impurity molecules including light chain-exchanged molecules (NPL 1). This document uses a method in which each molecule is separated into F(ab) and then subjected to mass spectrometry, and an estimated value of the content of each molecule is calculated by theoretical calculation based on the results of the mass spectrometry and on the probability of pairing between heavy and light chains. However, this method cannot accurately quantify light chain-exchanged molecules because it does not measure the contents directly from the analysis results, but merely obtains an estimated value by using theoretical calculation.

### [Citation List]

### [Patent Literature]

[PTL 1] WO96/27011
[PTL 2] WO2006/106905
[PTL 3] WO2013/065708
[PTL 4] WO2007/114325

### [Non-Patent Literature]

[NPL 1] Yin et.al.,MABS2016, VOL. 8, NO. 8, 1467-1476

### [Summary of Invention]

### [Technical Problem]

The invention of the present disclosure has been made under circumstances as described above. In one non-limiting embodiment, an objective of the present invention is to provide an analysis method for separately quantifying a multi-specific antigen-binding molecule of interest and a light chain-exchanged molecule comprised in a composition.

### [Solution to Problem]

In one non-limiting embodiment, as a result of dedicated studies, the present inventors discovered an analysis method for measuring the contents (content ratio) of a multi-specific antigen-binding molecule and a light chain-exchanged molecule comprised in a composition by using differences in combinations of F(ab) fragments comprised in multi-specific antigen-binding molecules and light chain-exchanged molecules. Specifically, the molecules comprised in the composition are treated to generate multiple types of F(ab) fragments, and the respective F(ab) fragments are separated by using, for example, their charge differences, and the content (content ratio) of the respective fragments is determined, thereby enabling to determine the presence or absence and the content or content ratio of light chain-exchanged molecules contained in the composition.

The present disclosure is based on these findings and, in particular, encompasses embodiments exemplified below.
[1] A method for analyzing a light chain-exchanged molecule in a composition comprising a multi-specific antigen-binding molecule,
   wherein the multi-specific antigen-binding molecule is a molecule (H1L1/H2L2) comprising a first F(ab) (H1L1) comprising a first heavy-chain variable domain and a first light-chain variable domain which are directed against a first antigen, and a second F(ab) (H2L2) comprising a second heavy-chain variable domain and a second light-chain variable domain which are directed against a second antigen;
   wherein the light chain-exchanged molecule is a molecule (H1L2/H2L1) comprising a third F(ab) (H1L2) comprising the first heavy-chain variable domain and the second light-chain variable domain, and a fourth F(ab) (H2L1) comprising the second heavy-chain variable domain and the first light-chain variable domain;
   wherein the analysis method comprises the following steps 1 to 2:
      1) treating the composition comprising the multi-specific antigen-binding molecule and thereby generating two or more types of F(ab) fragments; and
      2) measuring the two or more types of F(ab) fragments by a separation method based on electric charge or hydrophobic interaction and determining the content of the light chain-exchanged molecule in the composition, or the content ratio of the light chain-exchanged molecule to the multi-specific antigen-binding molecule in the composition.
[2] The method of [1], wherein step 1) comprises the following steps 1-1 and 1-2:
   1-1) cleaving the molecules comprised in the composition and thereby generating a F(ab)'2 fragment; and
   1-2) cleaving the F(ab)'2 fragment and thereby generating two or more types of F(ab) fragments.
[3] The method of [2], wherein step 1-1 is a step of cleaving with protease the Fc side of the hinge region of the molecules comprised in the composition.
[4] The method of [3], wherein the protease is any one of a bacteria-derived antibody-degrading enzyme, pepsin, and ficin, or a combination thereof.
[5] The method of any one of [2] to [4], wherein step 1-2 is a step of cleaving the disulfide bonds in the F(ab)'2 fragment with a reducing agent.
[6] The method of [5], wherein the reducing agent is any one of TCEP, 2-MEA, cysteine, dithiothreitol, 2-mercaptoethanol, 3-mercapto-1,2-propanediol, and TBP, or a combination thereof.
[7] The method of [1], wherein step 1 is a step of cleaving with protease the F(ab) side of the hinge region of the molecules comprised in the composition.
[8] The method of [7], wherein the protease is any one of a bacteria-derived antibody-degrading enzyme, papain, and Lys-C, or a combination thereof.
[9] The method of any one of [1] to [8], wherein step 2 is a step of measuring the two or more types of F(ab) fragments by any one of cation-exchange chromatography, anion-exchange chromatography, hydrophobic interaction chromatography, and reverse-phase chromatography, or a combination thereof.
[10] The method of any one of [1] to [9], wherein the two or more types of F(ab) fragments generated in step 1 are different in isoelectric point from each other.
[11] The method of any one of [1] to [10], wherein the multi-specific antigen-binding molecule (H1L1/H2L2) is a molecule in which an amino acid residue has been modified so as to provide an isoelectric point different from those of the following impurities (1) to (8):
   (1) a homodimer (H1L1/H1L1) of the first F(ab) (H1L1), which comprises the first heavy-chain variable domain and the first light-chain variable domain;
   (2) a homodimer (H2L2/H2L2) of the second F(ab) (H2L2), which comprises the second heavy-chain variable domain and the second light-chain variable domain;
   (3) a homodimer (H1L2/H1L2) of the third F(ab) (H1L2), which comprises the first heavy-chain variable domain and the second light-chain variable domain;
   (4) a homodimer (H2L1/H2L1) of the fourth F(ab) (H2L1), which comprises the second heavy-chain variable domain and the first light-chain variable domain;
   (5) a heterodimer (H1L1/H1L2) comprising the first F(ab) (H1L1), which comprises the first heavy-chain variable domain and the first light-chain variable domain, and the third F(ab) (H1L2), which comprises the first heavy-chain variable domain and the second light-chain variable domain;
   (6) a heterodimer (H1L1/H2L1) comprising the first F(ab) (H1L1), which comprises the first heavy-chain variable domain and the first light-chain variable domain, and the fourth F(ab) (H2L1), which comprises the second heavy-chain variable domain and the first light-chain variable domain;
   (7) a heterodimer (H2L2/H1L2) comprising the second F(ab) (H2L2), which comprises the second heavy-chain variable domain and the second light-chain variable domain, and the third F(ab) (H1L2), which comprises the first heavy-chain variable domain and the second light-chain variable domain; and
   (8) a heterodimer (H2L2/H2L1) comprising the second F(ab) (H2L2), which comprises the second heavy-chain variable domain and the second light-chain variable domain, and the fourth F(ab) (H2L1), which comprises the second heavy-chain variable domain and the first light-chain variable domain.
[12] The method of [11], wherein the composition comprising a multi-specific antigen-binding molecule has had impurities (1) to (8) removed through a purification step that uses the difference in isoelectric point.
[13] The method of any one of [1] to [12], wherein the multi-specific antigen-binding molecule is a bispecific antibody.
[14] The method of [13], wherein the bispecific antibody is an antibody in which at least one amino acid residue selected from the amino acid residues at positions 1, 3, 5, 8, 10, 12, 13, 15, 16, 19, 23, 25, 26, 39, 42, 43, 44, 46, 68, 71, 72, 73, 75, 76, 81, 82b, 83, 85, 86, 97, 105, 108, 110, and 112 according to Kabat numbering in the heavy-chain variable domain, and the amino acid residues at positions 137, 196, 203, 214, 217, 233, 268, 274, 276, 297, 355, 392, 419, and 435 according to EU numbering in the heavy-chain constant domain has been modified.
[15] The method of any one of [1] to [14], wherein the composition comprising a multi-specific antigen-binding molecule or a bispecific antibody is a pharmaceutical composition.
[16] The method of [13] or [14], wherein the bispecific antibody is an antibody comprising a F(ab) that binds to blood coagulation factor IX and/or activated blood coagulation factor IX, and a Fab that binds to blood coagulation factor X.
[17] A method for quality control of a pharmaceutical composition comprising a multi-specific antigen-binding molecule,
   wherein the multi-specific antigen-binding molecule is a molecule (H1L1/H2L2) comprising a first F(ab) (H1L1) comprising a first heavy-chain variable domain and a first light-chain variable domain which are directed against a first antigen, and a second F(ab) (H2L2) comprising a second heavy-chain variable domain and a second light-chain variable domain which are directed against a second antigen,
   wherein a light chain-exchanged molecule is a molecule (H1L2/H2L1) comprising a third F(ab) (H1L2) comprising the first heavy-chain variable domain and the second light-chain variable domain, and a fourth F(ab) (H2L1) comprising the second heavy-chain variable domain and the first light-chain variable domain,
   wherein the quality control method comprises the following steps 1-3:
      1) treating the composition comprising the multi-specific antigen-binding molecule and thereby generating two or more types of F(ab) fragments;
      2) measuring the two or more types of F(ab) fragments by a separation method based on electric charge or hydrophobic interaction and determining the content of the light chain-exchanged molecule in the composition or the content ratio of the light chain-exchanged molecule to the multi-specific antigen-binding molecule in the composition; and
      3) confirming that the content or content ratio of the light chain-exchanged molecule determined in step 2) is not more than the predetermined acceptable value for the content/content ratio.
[18] A method for quality evaluation of a pharmaceutical composition comprising a multi-specific antigen-binding molecule,
   wherein the multi-specific antigen-binding molecule is a molecule (H1L1/H2L2) comprising a first F(ab) (H1L1) comprising a first heavy-chain variable domain and a first light-chain variable domain which are directed against a first antigen, and a second F(ab) (H2L2) comprising a second heavy-chain variable domain and a second light-chain variable domain which are directed against a second antigen,
   wherein a light chain-exchanged molecule is a molecule (H1L2/H2L1) comprising a third F(ab) (H1L2) comprising the first heavy-chain variable domain and the second light-chain variable domain, and a fourth F(ab) (H2L1) comprising the second heavy-chain variable domain and the first light-chain variable domain,
   wherein the quality evaluation method comprises the following steps 1-3:
      1) treating the composition comprising the multi-specific antigen-binding molecule and thereby generating two or more types of F(ab) fragments;
      2) measuring the two or more types of F(ab) fragments by a separation method based on electric charge or hydrophobic interaction and determining the content of the light chain-exchanged molecule in the composition or the content ratio of the light chain-exchanged molecule to the multi-specific antigen-binding molecule in the composition; and
      3) confirming that the content or content ratio of the light chain-exchanged molecule determined in step 2) is not more than the predetermined acceptable value for the content/content ratio.
[19] The method of [17] or [18], wherein step 1) comprises the following steps 1-1 and 1-2:
   1-1) cleaving the molecules comprised in the composition and thereby generating a F(ab)'2 fragment; and
   1-2) cleaving the F(ab)'2 fragment and thereby generating two or more types of F(ab) fragments.
[20] The method of [19], wherein step 1-1 is a step of cleaving with protease the Fc side of the hinge region of the molecules comprised in the composition.
[21] The method of [20], wherein the protease is any one of a bacteria-derived antibody-degrading enzyme, pepsin, and ficin, or a combination thereof.
[22] The method of any one of [19] to [21], wherein step 1-2 is a step of cleaving the disulfide bonds in the F(ab)'2 fragment with a reducing agent.
[23] The method of [22], wherein the reducing agent is any one of TCEP, 2-MEA, cysteine, dithiothreitol, 2-mercaptoethanol, 3-mercapto-1,2-propanediol, and TBP, or a combination thereof.
[24] The method of [17] or [18], wherein step 1 is a step of cleaving with protease the F(ab) side of the hinge region of the molecules comprised in the composition.
[25] The method of [24], wherein the protease is any one of a bacteria-derived antibody-degrading enzyme, papain, and Lys-C, or a combination thereof.
[26] The method of any one of [17] to [25], wherein step 2 is a step of measuring the two or more types of F(ab) fragments by any one of cation-exchange chromatography, anion-exchange chromatography, hydrophobic interaction chromatography, and reverse-phase chromatography, or a combination thereof.
[27] The method of any one of [17] to [26], wherein the two or more types of F(ab) fragments generated in step 1 are different in isoelectric point from each other.
[28] The method of any one of [17] to [27], wherein the multi-specific antigen-binding molecule (H1L1/H2L2) is a molecule in which an amino acid residue has been modified so as to provide an isoelectric point different from those of the following impurities (1) to (8):
   (1) a homodimer (H1L1/H1L1) of the first F(ab) (H1L1), which comprises the first heavy-chain variable domain and the first light-chain variable domain;
   (2) a homodimer (H2L2/H2L2) of the second F(ab) (H2L2), which comprises the second heavy-chain variable domain and the second light-chain variable domain;
   (3) a homodimer (H1L2/H1L2) of the third F(ab) (H1L2), which comprises the first heavy-chain variable domain and the second light-chain variable domain;
   (4) a homodimer (H2L1/H2L1) of the fourth F(ab) (H2L1), which comprises the second heavy-chain variable domain and the first light-chain variable domain;
   (5) a heterodimer (H1L1/H1L2) comprising the first F(ab) (H1L1), which comprises the first heavy-chain variable domain and the first light-chain variable domain, and the third F(ab) (H1L2), which comprises the first heavy-chain variable domain and the second light-chain variable domain;
   (6) a heterodimer (H1L1/H2L1) comprising the first F(ab) (H1L1), which comprises the first heavy-chain variable domain and the first light-chain variable domain, and the fourth F(ab) (H2L1), which comprises the second heavy-chain variable domain and the first light-chain variable domain;
   (7) a heterodimer (H2L2/H1L2) comprising the second F(ab) (H2L2), which comprises the second heavy-chain variable domain and the second light-chain variable domain, and the third F(ab) (H1L2), which comprises the first heavy-chain variable domain and the second light-chain variable domain; and
   (8) a heterodimer (H2L2/H2L1) comprising the second F(ab) (H2L2), which comprises the second heavy-chain variable domain and the second light-chain variable domain, and the fourth F(ab) (H2L1), which comprises the second heavy-chain variable domain and the first light-chain variable domain.
[29] The method of [28], wherein the composition comprising a multi-specific antigen-binding molecule has had impurities (1) to (8) removed through a purification step that uses the difference in isoelectric point.
[30] The method of any one of [17] to [29], wherein the multi-specific antigen-binding molecule is a bispecific antibody.
[31] The method of [30], wherein the bispecific antibody is an antibody in which at least one amino acid residue selected from the amino acid residues at positions 1, 3, 5, 8, 10, 12, 13, 15, 16, 19, 23, 25, 26, 39, 42, 43, 44, 46, 68, 71, 72, 73, 75, 76, 81, 82b, 83, 85, 86, 97, 105, 108, 110, and 112 according to Kabat numbering in the heavy-chain variable domain, and the amino acid residues at positions 137, 196, 203, 214, 217, 233, 268, 274, 276, 297, 355, 392, 419, and 435 according to EU numbering in the heavy-chain constant domain has been modified.
[32] The method of any one of [17] to [31], wherein the composition comprising a multi-specific antigen-binding molecule or a bispecific antibody is a pharmaceutical composition.
[33] The method of [30] or [31], wherein the bispecific antibody is an antibody comprising a F(ab) that binds to blood coagulation factor IX and/or activated blood coagulation factor IX, and a F(ab) that binds to blood coagulation factor X.
[34] An active pharmaceutical ingredient whose quality is guaranteed by the quality control method or quality evaluation method of any one of [17] to [33].

### [Effects of the Invention]

In one non-limiting embodiment, the analysis method of the present disclosure demonstrates that the content of a light-chain exchanged molecule can be accurately measured directly from the results of measurements by using, for example, cation-exchange chromatography (CEX). This method is distinct from conventional analysis methods in which estimated values are obtained using theoretical calculation.

The analysis method of the present disclosure enables determination of whether a light-chain exchanged molecule is included as impurity in a composition comprising a multi-specific antigen-binding molecule of interest and, if it is included, accurate quantification of its content (content ratio). There is no limitation on the molecular form of a multi-specific antigen-binding molecule to be analyzed, and the method is applicable also to bispecific F(ab)'2 fragments, scFvs, and the like in addition to bispecific antibodies. Further, a multi-specific antigen-binding molecule that has two different F(ab)s, one of which has binding specificity to two or more antigens may be used.

### [Brief Description of Drawings]

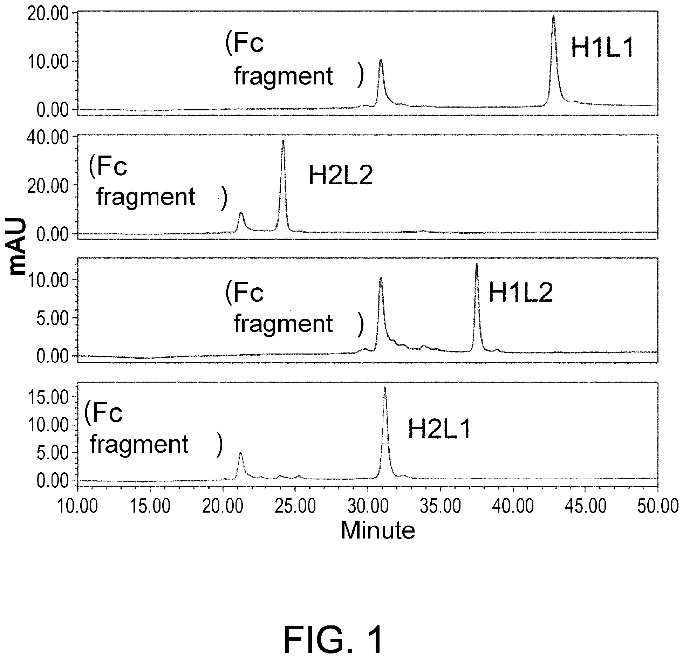

[Fig. 1] This figure shows the results of analysis in which a homodimer (H1L1/H1L1) consisting of heavy and light chains against blood coagulation factor IX and/or activated blood coagulation factor IX (FIX(a)), a homodimer (H2L2/H2L2) consisting of heavy and light chains against blood coagulation factor X (FX), a homodimer (H1L2/H1L2) consisting of heavy chains against blood coagulation factor IX and/or activated blood coagulation factor IX and light chains against blood coagulation factor X, and a homodimer (H2L1/H2L1) consisting of heavy chains against blood coagulation factor X and light chains against blood coagulation factor IX and/or activated blood coagulation factor IX were prepared and separated into four types of F(ab) fragments, H1L1, H2L2, H1L2, and H2L1, and then analyzed by CEX.

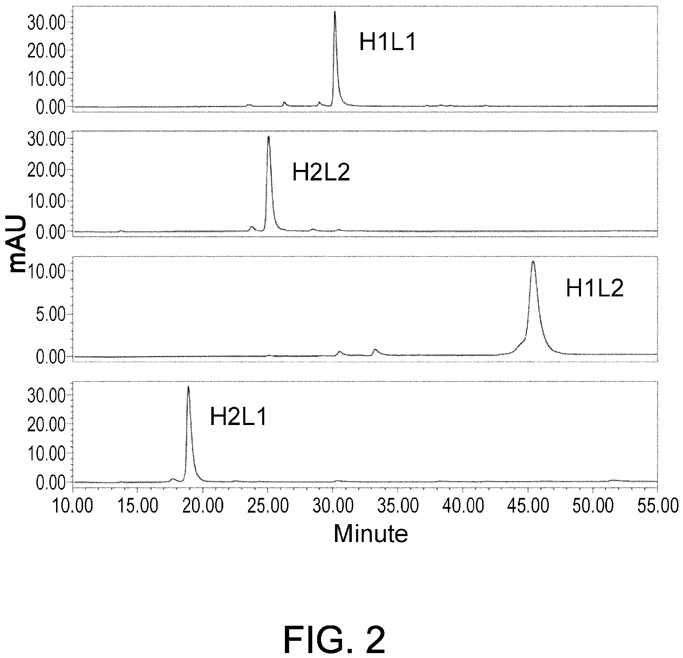

[Fig. 2] This figure shows the results of analysis in which a homodimer (H1L1/H1L1) consisting of heavy and light chains against epiregulin (EREG), a homodimer (H2L2/H2L2) consisting of heavy and light chains against GPC3, a homodimer (H1L2/H1L2) consisting of heavy chains against EREG and light chains against GPC3, and a homodimer (H2L1/H2L1) consisting of heavy chains against GPC3 and light chains against EREG were prepared and separated into four types of F(ab) fragments, H1L1, H2L2, H1L2, and H2L1, and then analyzed by CEX.

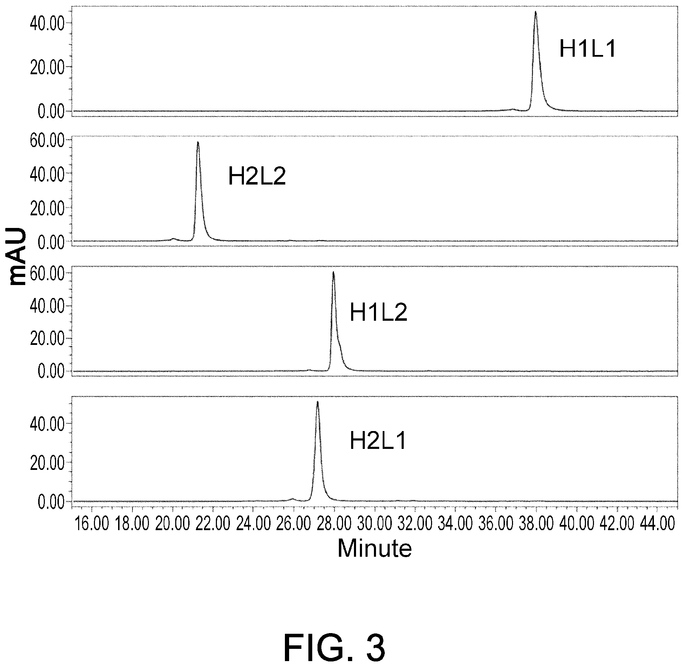

[Fig. 3] This figure shows the results of analysis in which a homodimer (H1L1/H1L1) consisting of heavy and light chains against IL-6 receptor (IL-6R), a homodimer (H2L2/H2L2) consisting of heavy and light chains against KLH, a homodimer (H1L2/H1L2) consisting of heavy chains against IL-6R and light chains against KLH, and a homodimer (H2L1/H2L1) consisting of heavy chains against KLH and light chains against IL-6R were prepared and separated into four types of F(ab) fragments, H1L1, H2L2, H1L2, and H2L1, and then analyzed by CEX.

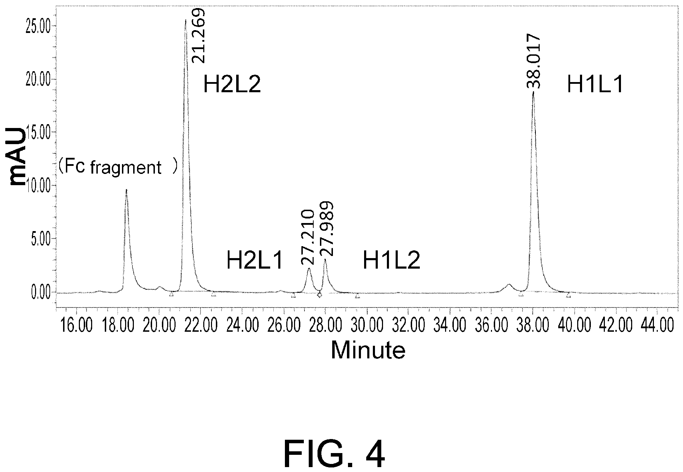

[Fig. 4] This figure shows the results of quantification of the amount of impurities contained in a mixed sample of anti-IL-6R antibody/anti-KLH antibody and their impurity molecules (light chain-exchanged molecules).
[Fig. 5] This figure shows schematic diagrams of a bispecific antibody of interest, its light chain-exchanged molecule, and other impurities (eight types) due to mispairing that can be generated during the process of producing a bispecific antibody with non-common light-chains. The bispecific antibody of interest has two different F(ab)s, indicated as H1L1 and H2L2 in the figure, and may be herein referred to as H1L1/H2L2. The light chain-exchanged molecule thereof has two different F(ab)s, indicated as H1L2 and H2L1 in the figure, and may be herein referred to as H1L2/H2L1. The combinations of two F(ab)s contained in the respective impurities due to other mispairing differ from the combination contained in the bispecific antibody of interest and the combination contained in its light chain-exchanged molecule. Given in brackets above the schematic diagram of each molecule is the theoretical pI (isoelectric point) value for each molecule for the case where the anti-FIX(a)/anti-FX bispecific antibody shown in Table 1 is the bispecific antibody of interest.

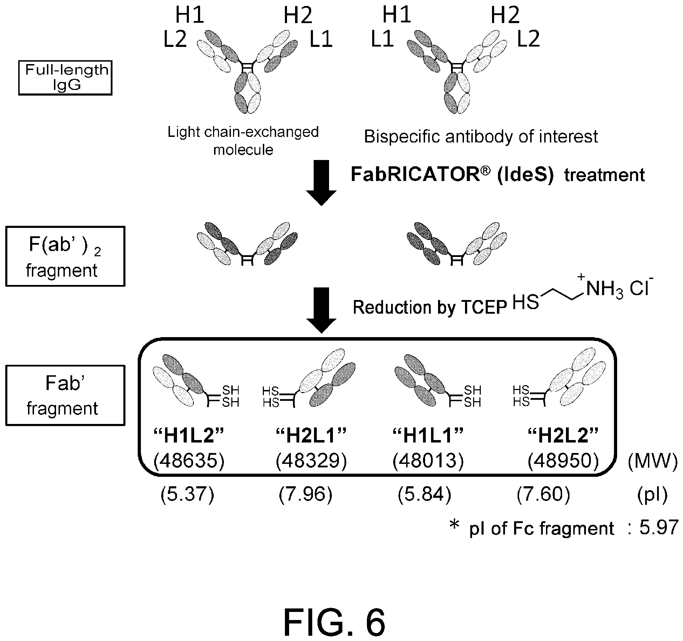

[Fig. 6] This figure shows a schematic diagram of an exemplary method for generating four types of F(ab) fragments from a molecule of interest and its light chain-exchanged molecule by a two-step treatment. Given in brackets below each F(ab) fragment are the molecular weight (MW) and the theoretical pI value for each fragment for the case where the anti-FIX(a)/anti-FX bispecific antibody shown in Table 1 is the bispecific antibody of interest.

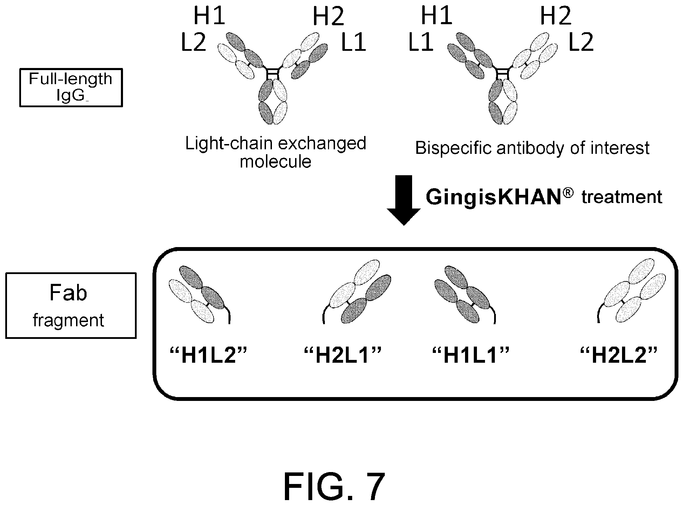

[Fig. 7] This figure shows a schematic diagram of an exemplary method for generating four types of F(ab) fragments from a bispecific antibody of interest and its light chain-exchanged molecule by a single-step treatment.

### [Description of Embodiments]

### I. Definitions

### Antigen-binding molecule

Herein, the term "antigen-binding molecule" refers, in its broadest sense, to a molecule that specifically binds to an antigenic determinant (epitope). In one embodiment, the antigen-binding molecule is an antibody, antibody fragment, or antibody derivative. In one embodiment, the antigen-binding molecule is a non-antibody protein, or a fragment thereof, or a derivative thereof.

In the present specification, "specifically binds" means binding in a state where one of the molecules involved in specific binding does not show any significant binding to molecules other than a single or a number of binding partner molecules. Furthermore, it is also used when an antigen-binding domain is specific to a particular epitope among multiple epitopes contained in an antigen. When an epitope bound by an antigen-binding domain is contained in multiple different antigens, antigen-binding molecules comprising the antigen-binding domain can bind to various antigens that have the epitope.

In the present disclosure, the recitation "binds to the same epitope" means that the epitopes to which two antigen-binding domains bind at least partially overlap each other. The degree of the overlap is, but not limited to, at least 10% or more, preferably 20% or more, 30% or more, 40% or more, 50% or more, 60% or more, 70% or more, and 80% or more, particularly preferably 90% or more, and most preferably 100%.

### Antibody

The term "antibody" herein is used in the broadest sense and encompasses various antibody structures, including but not limited to monoclonal antibodies, polyclonal antibodies, multi-specific antibodies (e.g., bispecific antibodies), and antibody fragments so long as they exhibit the desired antigen-binding activity.

"Binding activity" refers to the strength of the sum total of noncovalent interactions between one or more binding sites of a molecule (e.g., an antibody) and its binding partner (e.g., an antigen). Herein, binding activity is not strictly limited to a 1:1 interaction between members of a binding pair (e.g., antibody and antigen). For example, when the members of a binding pair reflect a monovalent 1:1 interaction, the binding activity refers to the intrinsic binding affinity (affinity). When a member of a binding pair is capable of both monovalent binding and multivalent binding, the binding activity is the sum of each binding strength. The binding activity of a molecule X for its partner Y can generally be represented by the dissociation constant (KD) or "amount of bound analyte per unit amount of ligand". Binding activity can be measured by common methods known in the art, including those described herein.

In one aspect, the antigen-binding molecules and antibodies analyzed by the method of the present disclosure may be tested regarding their antigen-binding activity by known methods such as ELISA and Western blot.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The terms "full length antibody", "intact antibody", and "whole antibody" are used herein interchangeably to refer to an antibody having a structure substantially similar to a native antibody structure or having heavy chains that contain an Fc region as defined herein.

Herein, "antigen-binding domain" refers to a region that specifically binds and is complementary to the whole or a portion of an antigen. Herein, an antigen-binding molecule comprises an antigen-binding domain. When the molecular weight of an antigen is large, an antigen-binding domain can only bind to a particular portion of the antigen. The particular portion is called "epitope". In one embodiment, an antigen-binding domain comprises an antibody fragment which binds to a particular antigen. An antigen-binding domain can be provided from one or more antibody "variable domains". In a non-limiting embodiment, the antigen-binding domains comprise both the antibody light chain variable region (VL) and antibody heavy chain variable region (VH). Examples of such antigen-binding domains include "single-chain Fv (scFv)", "single-chain antibody", "Fv", "single-chain Fv2 (scFv2)", "Fab", and "Fab‴. In other embodiments, an antigen-binding domain comprises a non-antibody protein which binds to a particular antigen, or a fragment thereof. In a specific embodiment, an antigen-binding domain comprises a hinge region.

### Variable domain

The term "variable region" or "variable domain" refers to the domain of an antibody heavy or light chain that is involved in binding the antibody or antigen-binding molecule to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

### Constant region

In one embodiment of the present invention, constant regions are preferably antibody constant regions, more preferably IgG1, IgG2, IgG3, and IgG4-type antibody constant regions, and even more preferably human IgG1, IgG2, IgG3, and IgG4-type antibody constant regions. Furthermore, in another embodiment of the present invention, constant regions are preferably heavy chain constant regions, more preferably IgG1, IgG2, IgG3, and IgG4-type heavy chain constant regions, and even more preferably human IgG1, IgG2, IgG3, and IgG4-type heavy chain constant regions. The amino acid sequences of the human IgG1 constant region, the human IgG2 constant region, the human IgG3 constant region, and the human IgG4 constant region are known. For the constant regions of human IgG1, human IgG2, human IgG3, and human IgG4, a plurality of allotype sequences with genetic polymorphism are described in Sequences of proteins of immunological interest, NIH Publication No.91-3242, and any of them can be used in the present invention. Amino acid-modified constant regions of the present invention may contain other amino acid mutations or modifications, as long as they include an amino acid mutation of the present invention.

The term "hinge region" denotes an antibody heavy chain polypeptide portion in a wild-type antibody heavy chain that joins the CH1 domain and the CH2 domain, e.g., from about position 216 to about position 230 according to the EU numbering system, or from about position 226 to about position 243 according to the Kabat numbering system. It is known that in a native IgG antibody, cysteine residue at position 220 according to EU numbering in the hinge region forms a disulfide bond with cysteine residue at position 214 in the antibody light chain. It is also known that between the two antibody heavy chains, disulfide bonds are formed between cysteine residues at position 226 and between cysteine residues at position 229 according to EU numbering in the hinge region. A hinge region herein includes wild-type hinge regions as well as variants in which amino acid residue(s) in a wild-type hinge region is altered by substitution, addition, or deletion.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

### Antibody fragment

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); single chain Fabs (scFabs); single domain antibodies; and multi-specific antibodies formed from antibody fragments.

### Variable fragment (Fv)

Herein, the term "variable fragment (Fv)" refers to the minimum unit of an antibody-derived antigen-binding domain that is composed of a pair of the antibody light chain variable region (VL) and antibody heavy chain variable region (VH). In 1988, Skerra and Pluckthun found that homogeneous and active antibodies can be prepared from the *E. coli* periplasm fraction by inserting an antibody gene downstream of a bacterial signal sequence and inducing expression of the gene in *E. coli* (Science (1988) 240(4855), 1038-1041). In the Fv prepared from the periplasm fraction, VH associates with VL in a manner so as to bind to an antigen.

### scFv, single-chain antibody, and sc(Fv)2

Herein, the terms "scFv", "single-chain antibody", and "sc(Fv)2" all refer to an antibody fragment of a single polypeptide chain that contains variable regions derived from the heavy and light chains, but not the constant region. In general, a single-chain antibody also contains a polypeptide linker between the VH and VL domains, which enables formation of a desired structure that is thought to allow antigen binding. The single-chain antibody is discussed in detail by Pluckthun in "The Pharmacology of Monoclonal Antibodies, Vol. 113, Rosenburg and Moore, eds., Springer-Verlag, New York, 269-315 (1994)". See also International Patent Publication WO 1988/001649; US Patent Nos. 4,946,778 and 5,260,203. In a particular embodiment, the single-chain antibody can be bispecific and/or humanized.

scFv is an antigen-binding domain in which VH and VL forming Fv are linked together by a peptide linker (Proc. Natl. Acad. Sci. U.S.A. (1988) 85(16), 5879-5883). VH and VL can be retained in close proximity by the peptide linker.

sc(Fv)2 is a single-chain antibody in which four variable regions of two VL and two VH are linked by linkers such as peptide linkers to form a single chain (J Immunol. Methods (1999) 231(1-2), 177-189). The two VH and two VL may be derived from different monoclonal antibodies. Such sc(Fv)2 preferably includes, for example, a bispecific sc(Fv)2 that recognizes two epitopes present in a single antigen as disclosed in the Journal of Immunology (1994) 152(11), 5368-5374. sc(Fv)2 can be produced by methods known to those skilled in the art. For example, sc(Fv)2 can be produced by linking scFv by a linker such as a peptide linker.

Herein, the form of an antigen-binding domain forming an sc(Fv)2 include an antibody in which the two VH units and two VL units are arranged in the order of VH, VL, VH, and VL ([VH]-linker-[VL]-linker-[VH]-linker-[VL]) beginning from the N terminus of a single-chain polypeptide. The order of the two VH units and two VL units is not limited to the above form, and they may be arranged in any order. Example order of the form is listed below.
[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

### F(ab)

In the present specification, "F(ab) (also referred to as Fab or Fab')" is composed of a light chain consisting of VL (light chain variable region) and CL (light chain constant region) and a portion consisting of a heavy chain VH (heavy chain variable region) and CH1 (γ1 region in the heavy chain constant region), and may take the structure wherein the portion of the heavy chain and the light chain are bound by a disulfide bond at the C terminal region. Further, F(ab) may comprise a portion of the hinge region. F(ab) as an antibody fragment may be simply referred to as F(ab) or F(ab) fragment. F(ab) as one part of an antigen-binding molecule (e.g., antibody) may also be referred to simply as F(ab), and also as F(ab) portion or F(ab) region to distinguish from F(ab) fragments.

In the present specification, F(ab) may or may not have antigen-binding activity. In one illustrative embodiment, the two types of F(ab)s comprised in a multi-specific antigen-binding molecule (H1L1/H2L2) both have antigen-binding activity. In one illustrative embodiment, the antigen-binding activity of one or both of the two types of F(ab)s comprised in the light chain-exchanged molecule (H1L2/H2L1) of a multi-specific antigen-binding molecule may be weaker than the antigen-binding activity of the two types of F(ab)s comprised in the multi-specific antigen-binding molecule. In a specific embodiment, one or both of the two types of F(ab)s comprised in the light chain-exchanged molecule of a multi-specific antigen-binding molecule do not have antigen-binding activity.

### F(ab')2

In the present specification, "F(ab')2 (also referred to as F(ab)'2)" refers to an antibody fragment having the structure of two F(ab)s bound together (also referred to as F(ab')2 fragment), or to one part of an antigen-binding molecule (also referred to as F(ab')2 portion or F(ab')2 region). Binding of the two F(ab)s in F(ab')2 is, for example, a disulfide bond at the immunoglobulin hinge region, but is not limited thereto. In one embodiment, F(ab')2 comprises a portion of the hinge region. In one embodiment, F(ab')2 comprises two light chains and two heavy chain portions comprising the constant region of the CH1 domain and one part of the CH2 domain, so that a disulfide bond is formed between the two heavy chain portions.

In the present specification, F(ab')2 may or may not have antigen-binding activity. In one illustrative embodiment, the F(ab')2 comprised in a multi-specific antigen-binding molecule all have antigen-binding activity. In one illustrative embodiment, the antigen-binding activity of a F(ab')2 comprised in the light chain-exchanged molecule of a multi-specific antigen-binding molecule is weaker than the antigen-binding activity of a F(ab')2 comprised in the multi-specific antigen-binding molecule. In a specific embodiment, F(ab')2 comprised in the light chain-exchanged molecule of a multi-specific antigen-binding molecule does not have antigen-binding activity.

### Multi-specific antigen-binding molecule

In certain embodiments, an antigen-binding molecule provided herein is a multi-specific antigen-binding molecule, e.g. a bispecific antigen-binding molecule. Multi-specific antigen-binding molecules are monoclonal antigen-binding molecules that have binding specificities for at least two different sites. In certain embodiments, one of the binding specificities is for a particular antigen (e.g., blood coagulation factor IX (FIX), epiregulin (EREG), IL-6 receptor (IL-6R), etc.) and the other is for any other antigen (e.g., blood coagulation factor X (FX), GPC3, KLH, etc.). In certain embodiments, multi-specific antigen-binding molecules may bind to two different epitopes on a single antigen. Further, in a specific embodiment, a multi-specific antigen-binding molecule comprises one antigen-binding site having binding specificity to two or more different antigens. As one example, a multi-specific antigen-binding molecule comprises a first antigen-binding site against two or more different antigens and a second antigen-binding site against one antigen. The multi-specific antigen-binding molecule is a molecule having two different antigen-binding sites and having binding specificity against three or more different antigens. Multi-specific antigen-binding molecules can be prepared as full length antibodies or antibody fragments.

Techniques for making multi-specific antigen-binding molecules include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antigen-binding molecules may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bispecific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g. Gruber et al., J. Immunol., 152:5368 (1994)); and preparing trispecific antibodies as described, e.g., in Tutt et al. J. Immunol. 147: 60 (1991).

### Generation of F(ab) fragments and F(ab')2 fragments by treating a composition

In a particular embodiment, F(ab) fragments are generated by treating a composition containing a multi-specific antigen-binding molecule provided herein. In the embodiment, "a composition is 'treated‴ or "'treating' a composition" means that a treatment is conducted on a composition for generating F(ab)s by cleaving a multi-specific antigen-binding molecule and/or a light chain-exchanged molecule, and the treatment can be implemented using a method known to one skilled in the art. "Cleaving" a molecule refers to, for example, dissociating the binding between amino acid residues or disulfide bonds in the molecule.

As a method for the treatment, for example, the multi-specific antigen-binding molecule and/or light chain-exchanged molecule can be cleaved by adding a protease to the composition. Proteases such as bacteria-derived antibody-degrading enzymes represented by GingisKHAN (registered trademark) (Kgp), FabRICATOR (registered trademark) (IdeS), FabRICATOR (registered trademark)Z (IdeZ), FabALACTICA (registered trademark) (IgdE), and FabULOUS (registered trademark) (SpeB) (all by Genovis), pepsin, papain, and Lys-C may be used.

The protease treatment may cleave the Fc side or the F(ab) side of the hinge region of the multi-specific antigen-binding molecule and/or light chain-exchanged molecule.

In the case of cleavage at the F(ab) side, F(ab) fragments are generated by the cleavage (see Fig. 7). After the F(ab) fragments are thus generated, the Fc fragments are adsorbed to a Protein A column and removed, and F(ab) fragments can be obtained.

In contrast, in the case of cleavage at the Fc side, F(ab')2 fragments are generated (see Fig. 6). F(ab')2 fragments may also be obtained by reaction of the antibody with ficin under the conditions for generating the F(ab')2 fragments. After antigen-binding molecules (*e.g.,* complete monoclonal antibody) are partially digested with these proteases, the Fc fragments are adsorbed to a Protein A column and removed, and F(ab')2 fragments can be obtained.

Subsequently, F(ab) fragments (also referred to as Fab' fragments) can be generated by cleaving the disulfide bonds in the F(ab')2 fragments with a reducing agent (see Fig. 6). As the reducing agent, TCEP, 2-MEA, Cysteine, Dithiothreitol, 2-Mercaptoethanol, 3-mercapto-1,2-propanediol, TBP, and such can be used.

The terms "host cell", "host cell line", and "host cell culture" are used interchangeably and refer to cells into which exogenous nucleic acid has been introduced, including the progeny of such cells. Host cells include "transformants" and "transformed cells", which include the primary transformed cell and progeny derived therefrom without regard to the number of passages. Progenies may not be completely identical in nucleic acid content to a parent cell, and may contain mutations. Mutant progenies that have the same function or biological activity as screened or selected for in the originally transformed cell are included herein.

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

### Recombinant Methods and Compositions

Antigen-binding molecules may be produced using recombinant methods and compositions, e.g., as described in U.S. Patent No. 4,816,567. In a non-limiting example of such production, isolated nucleic acid encoding an antigen-binding molecule is used. Such nucleic acid may encode an amino acid sequence comprising the VL and/or an amino acid sequence comprising the VH of the antigen-binding molecule (e.g., the light and/or heavy chains of the antigen-binding molecule). One or more vectors (e.g., expression vectors) comprising such nucleic acid may be used. In an illustrative embodiment, a host cell comprising such nucleic acid or vector is used. In one such embodiment, a host cell comprises (e.g., has been transformed with): (1) a vector comprising a nucleic acid that encodes an amino acid sequence comprising the VL of the antigen-binding molecule and an amino acid sequence comprising the VH of the antigen-binding molecule, or (2) a first vector comprising a nucleic acid that encodes an amino acid sequence comprising the VL of the antigen-binding molecule and a second vector comprising a nucleic acid that encodes an amino acid sequence comprising the VH of the antigen-binding molecule. In one embodiment, the host cell is eukaryotic cell, e.g. a Chinese Hamster Ovary (CHO) cell or lymphoid cell (e.g., Y0, NS0, Sp2/0 cell). In one embodiment, a method of making an antigen-binding molecule is provided, wherein the method comprises culturing a host cell comprising a nucleic acid encoding the antigen-binding molecule, as provided above, under conditions suitable for expression of the antigen-binding molecule, and optionally recovering the antigen-binding molecule from the host cell (or host cell culture medium).

For recombinant production of an antigen-binding molecule, nucleic acid encoding an antigen-binding molecule is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antigen-binding molecule).

Suitable host cells for cloning or expression of antigen-binding molecule-encoding vectors include prokaryotic or eukaryotic cells described herein. For example, antigen-binding molecules may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli.*) After expression, the antigen-binding molecule may be isolated from the bacterial cell paste in a soluble fraction and can be further purified.

In addition to prokaryotes, eukaryotic microbes such as filamentous fungi or yeast are suitable cloning or expression hosts for antigen-binding molecule-encoding vectors, including fungi and yeast strains whose glycosylation pathways have been "humanized", resulting in the production of an antigen-binding molecule with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antigen-binding molecule are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See,* e.g., US Patent Nos. 5,959,177, 6,040,498, 6,420,548, 7,125,978, and 6,417,429 (describing PLANTIBODIES (trademark) technology for producing antigen-binding molecules in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antigen-binding molecule production, see, e.g., Yazaki and Wu, Methods in Molecular Biology, Vol. 248 (B.K.C. Lo, ed., Humana Press, Totowa, NJ), pp. 255-268 (2003).

### Multi-specific antigen-binding molecule (H1L1/H2L2) and its light chain-exchanged molecule (H1L2/H2L1)

A first F(ab) comprising or consisting of a first heavy-chain variable domain (VH comprised in H1) and a first light-chain variable domain (VL comprised in L1) is herein referred to as H1L1, and a second F(ab) comprising or consisting of a second heavy-chain variable domain (VH comprised in H2) and a second light-chain variable domain (VL comprised in L2) is herein referred to as H2L2, and a multi-specific antigen-binding molecule comprising H1L1 and H2L2 is herein referred to as H1L1/H2L2. A third F(ab) comprising or consisting of the first heavy-chain variable domain (VH comprised in H1) and the second light-chain variable domain (VL comprised in L2) is referred to as H1L2, and a fourth F(ab) comprising or consisting of the second heavy-chain variable domain (VH comprised in H2) and the first light-chain variable domain (VL comprised in L1) is referred to as H2L1, and a molecule comprising H1L2 and H2L1, namely a light chain-exchanged molecule of H1L1/H2L2 is referred to as H1L2/H2L1. Thus, the light chain-exchanged molecules described herein are impurity molecules generated due to inverse pairing (mispairing) between two different heavy chains and two different light chains that constitute a multi-specific antigen-binding molecule of interest.

In a non-limiting embodiment, light chain-exchanged molecule H1L2/H2L1 may be comprised as an impurity in a composition comprising multi-specific antigen-binding molecule H1L1/H2L2.

In one embodiment, the multi-specific antigen-binding molecule H1L1/H2L2 comprises: a first F(ab) (referred to as H1L1) comprising or consisting of a heavy chain portion (H1) comprising or consisting of a first heavy-chain variable (VH) domain and a CH1 domain, and a light chain portion (L1) comprising or consisting of a first light-chain variable (VL) domain and a CL domain; and a second F(ab) (referred to as H2L2) comprising or consisting of a heavy chain portion (H2) comprising or consisting of a second heavy-chain variable (VH) domain and a CH1 domain, and a light chain portion (L2) comprising or consisting of a second light-chain variable (VL) domain and a CL domain.

In one embodiment, the light chain-exchanged molecule H1L2/H2L1 of multi-specific antigen-binding molecule H1L1/H2L2 comprises: a third F(ab) (referred to as H1L2) comprising or consisting of a heavy chain portion (H1) comprising or consisting of the first heavy-chain variable (VH) domain and the CH1 domain, and a light chain portion (L2) comprising or consisting of the second light-chain variable (VL) domain and the CL domain; and a fourth F(ab) (referred to as H2L1) comprising or consisting of a heavy chain portion (H2) comprising or consisting of the second heavy-chain variable (VH) domain and the CH1 domain, and a light chain portion (L1) comprising or consisting of the first light-chain variable (VL) domain and the CL domain.

The first F(ab) H1L1 and the second F(ab) H2L2 may each have or not have antigen-binding activity. In a preferred exemplary embodiment, both H1L1 and H2L2 have antigen-binding activity.

The third F(ab) H1L2 and the fourth F(ab) H2L1 may each have or not have antigen-binding activity. In a non-limiting embodiment, either one or both of H1L2 and H2L1 have antigen-binding activity. In an exemplary embodiment, the antigen-binding activity of either one or both of H1L2 and H2L1 is lower compared to the antigen-binding activity of either one or both of H1L1 and H2L2. In a particular embodiment, either one or both of H1L2 and H2L1 do not have antigen-binding activity.

### Methods for analyzing a composition comprising a multi-specific antigen-binding molecule and light chain-exchanged molecule thereof

In one aspect, the present disclosure provides a method for analyzing an impurity molecule (*e.g.,* light chain-exchanged molecule) in a composition comprising a multi-specific antigen-binding molecule. In one embodiment of this aspect, the present disclosure provides a method for detecting an impurity molecule (*e.g.,* light chain-exchanged molecule) in a composition comprising a multi-specific antigen-binding molecule. In another embodiment, the present disclosure provides a method for measuring the content and/or content ratio of impurity molecules (*e.g.,* light chain-exchanged molecules) in a composition comprising a multi-specific antigen-binding molecule.

In another aspect, the present disclosure provides a method for analyzing a multi-specific antigen-binding molecule and an impurity molecule in a composition comprising a multi-specific antigen-binding molecule and an impurity molecule (*e.g.,* light chain-exchanged molecule). In one embodiment of this aspect, the present disclosure provides a method for detecting an impurity molecule in a composition comprising a multi-specific antigen-binding molecule and an impurity molecule (*e.g.,* light chain-exchanged molecule). In one embodiment of this aspect, the present disclosure provides a method for measuring the content and/or content ratio of multi-specific antigen-binding molecules and impurity molecules in a composition comprising a multi-specific antigen-binding molecule and an impurity molecule (*e.g.,* light chain-exchanged molecule). In another embodiment, the present disclosure provides a method for measuring the content and/or content ratio of multi-specific antigen-binding molecules in a composition comprising a multi-specific antigen-binding molecule and an impurity molecule (*e.g.,* light chain-exchanged molecule). In another embodiment, the present disclosure provides a method for measuring the purity of a multi-specific antigen-binding molecule in a composition comprising a multi-specific antigen-binding molecule and an impurity molecule (*e.g.,* light chain-exchanged molecule).

In one embodiment, the method of the present disclosure includes the following steps 1-2:
1) treating a composition comprising a multi-specific antigen-binding molecule and thereby generating a plurality of F(ab) fragments; and
2) measuring the plurality of F(ab) fragments by a separation method based on electric charge or hydrophobic interaction and determining the content of a light chain-exchanged molecule in the composition or the content ratio of the light chain-exchanged molecule to the multi-specific antigen-binding molecule in the composition.

In a particular embodiment, step 1 is a step of cleaving, with a protease, the F(ab) side of the hinge region of the molecules comprised in the composition. In a particular embodiment, step 1 includes generating F(ab) fragments by a treatment using a protease that cleaves the F(ab) region side of the immunoglobulin hinge region (the hinge portion of the antigen-binding molecule) under conditions for generating F(ab) fragments. Non-limiting examples of such protease may include papain, Lys-C, GingisKHAN (registered trademark) (Genovis), FabALACTICA (registered trademark) (Genovis), and ficin.

In another particular embodiment, step 1 includes the following steps 1-1 and 1-2:
1-1) cleaving the molecules comprised in the composition and thereby generating F(ab)'2 fragments; and
1-2) cleaving the F(ab)'2 fragments and thereby generating two or more types of F(ab) fragments.

In a particular embodiment, step 1-1 is a step of cleaving, with a protease, the Fc side of the hinge region of the molecules comprised in the composition. In a particular embodiment, step 1-1 includes a treatment using a protease that cleaves the Fc region side of the immunoglobulin hinge region (the hinge portion of the antigen-binding molecule) under conditions for generating F(ab)'2 fragments. Non-limiting examples of such protease may include pepsin, FabRICATOR (registered trademark) (Genovis), FabRICATOR (registered trademark)Z (Genovis), FabULOUS (registered trademark) (Genovis), and ficin.

In a particular embodiment, step 1-2 is a step of cleaving the disulfide bonds in the F(ab)'2 fragments with a reducing agent. Non-limiting examples of such a reducing agent may include TCEP, 2-MEA, Cysteine, Dithiothreitol, 2-Mercaptoethanol, 3-mercapto-1,2-propanediol, and TBP. In one embodiment, treatment with the reducing agent is carried out under conditions for cleaving the disulfide bonds in the hinge portion of F(ab)'2 fragments (immunoglobulin hinge region), and such conditions are known to one skilled in the art.

In a particular embodiment, step 2 can be carried out by a separation method based on electric charge or hydrophobic interaction, which is known to one skilled in the art. Such a separation method includes, but is not limited to, cation-exchange (CEX) chromatography, anion-exchange (AEX) chromatography, hydrophobic interaction chromatography (HIC), and reverse-phase chromatography.

In a particular embodiment, the two or more types of F(ab) fragments generated in step 1 are different in isoelectric point from each other. In one embodiment, the F(ab) fragments of H1L1, H2L2, H1L2, and H2L1 are constituted such that the F(ab) fragments are different in isoelectric point from each other due to modification of at least one amino acid residue that constitutes them.

In a particular embodiment, the multi-specific antigen-binding molecule (H1L1/H2L2) is a molecule in which amino acid residues have been modified so as to provide the molecule with a different isoelectric point from those of the following impurities (1) to (8):
(1) a homodimer (H1L1/H1L1) of the first F(ab) (H1L1), which comprises the first heavy-chain variable domain and the first light-chain variable domain;
(2) a homodimer (H2L2/H2L2) of the second F(ab) (H2L2), which comprises the second heavy-chain variable domain and the second light-chain variable domain;
(3) a homodimer (H1L2/H1L2) of the third F(ab) (H1L2), which comprises the first heavy-chain variable domain and the second light-chain variable domain;
(4) a homodimer (H2L1/H2L1) of the fourth F(ab) (H2L1), which comprises the second heavy-chain variable domain and the first light-chain variable domain;
(5) a heterodimer (H1L1/H1L2) comprising the first F(ab) (H1L1), which comprises the first heavy-chain variable domain and the first light-chain variable domain, and the third F(ab) (H1L2), which comprises the first heavy-chain variable domain and the second light-chain variable domain;
(6) a heterodimer (H1L1/H2L1) comprising the first F(ab) (H1L1), which comprises the first heavy-chain variable domain and the first light-chain variable domain, and the fourth F(ab) (H2L1), which comprises the second heavy-chain variable domain and the first light-chain variable domain;
(7) a heterodimer (H2L2/H1L2) comprising the second F(ab) (H2L2), which comprises the second heavy-chain variable domain and the second light-chain variable domain, and the third F(ab) (H1L2), which comprises the first heavy-chain variable domain and the second light-chain variable domain; and
(8) a heterodimer (H2L2/H2L1) comprising the second F(ab) (H2L2), which comprises the second heavy-chain variable domain and the second light-chain variable domain, and the fourth F(ab) (H2L1), which comprises the second heavy-chain variable domain and the first light-chain variable domain.

In a particular embodiment, impurities (1) to (8) are removed from a composition comprising the multi-specific antigen-binding molecule by a purification step that utilizes the difference in isoelectric points.

In a particular embodiment, the multi-specific antigen-binding molecule is a bispecific antibody. In one embodiment, the bispecific antibody comprises first and second heavy chains having an isoelectric point (pI) difference. Such heavy chains include heavy chain variable domains having a pI difference and/or heavy chain constant domains having a pI difference.

Heavy-chain constant domains having a pI difference may include an antibody heavy-chain constant region having a pI difference. The heavy-chain constant regions of IgG1, IgG2, IgG3, and IgG4, which intrinsically have difference in pI, may be used to introduce a pI difference to the first and second heavy chains. Alternatively, the amino acids in the heavy chain constant regions of the first heavy chain and the second heavy chain that cause differences in isoelectric point among these subclasses can be modified alone, or in combination with adjacent amino acids that do not have any effect on the isoelectric points to generate non-wild-type human constant regions, and pI difference can be introduced into the two constant regions. The modification sites for introducing a pI difference to the constant regions include, for example, 137th, 196th, 203rd, 214th, 217th, 233rd, 268th, 274th, 276th, 297th, 355th, 392nd, 419th, and 435th position of the H chain, according to EU numbering, in the heavy chain constant region. In addition, the 297th position, a glycosylation site, may be included in the modification site for introducing a pI difference, since removal of a sugar chain in the heavy chain constant region may cause a pI difference.

Similarly for heavy chain variable domains, it is possible to introduce a pI difference to the two heavy chain variable domains by modifying amino acid residues at particular positions in the heavy chain variable domains in the first and second heavy chains. Such heavy chain variable domains having a pI difference include, for example, heavy chain variable domains in which the amino acid residue at a particular position in the first heavy chain variable domain has a charge while the amino acid residue at the corresponding position in the second heavy chain variable domain does not have a charge or has an opposite charge to that of the amino acid residue at the corresponding position in the first heavy chain variable domain.

In a particular embodiment, the bispecific antibody is an antibody in which at least one amino acid residue selected from the amino acid residues at positions 1, 3, 5, 8, 10, 12, 13, 15, 16, 19, 23, 25, 26, 39, 42, 43, 44, 46, 68, 71, 72, 73, 75, 76, 81, 82b, 83, 85, 86, 97, 105, 108, 110, and 112 according to Kabat numbering in the heavy-chain variable domain, and the amino acid residues at positions 137, 196, 203, 214, 217, 233, 268, 274, 276, 297, 355, 392, 419, and 435 according to EU numbering in the heavy-chain constant domain has been modified. In a particular embodiment, the bispecific antibody is an antibody comprising a F(ab) that binds to blood coagulation factor IX and/or activated blood coagulation factor IX, and a F(ab) that binds to blood coagulation factor X.

In a particular embodiment, the composition comprising the multi-specific antigen-binding molecule or the bispecific antibody is a pharmaceutical composition.

In a particular embodiment, the method of the present disclosure is a method of controlling quality (or a method of evaluating quality) of a pharmaceutical composition comprising the following steps 1 to 3:
1) treating a composition comprising a multi-specific antigen-binding molecule and thereby generating a plurality of F(ab) fragments;
2) measuring the plurality of F(ab) fragments by a separation method based on electric charge or hydrophobic interaction and determining the content of a light chain-exchanged molecule in the composition or the content ratio of the light chain-exchanged molecule to the multi-specific antigen-binding molecule in the composition; and
3) confirming that the content or content ratio of the light chain-exchanged molecule determined in step 2) is not more than the predetermined acceptable value for the content/content ratio of an impurity molecule.

In a particular embodiment, the present disclosure provides an active pharmaceutical ingredient whose quality is guaranteed by the above-described quality control method or quality evaluation method.

All of the prior art documents cited in this specification are herein incorporated by reference.

### [Example]

The present invention is specifically explained below with reference to Examples, but it is not to be construed as being limited thereto.

### F(ab)s of a multi-specific antigen-binding molecule and its light-chain exchanged molecule

In the present Example, a first F(ab) comprising a heavy chain portion (H1) comprising a first heavy-chain variable (VH) domain and a CH1 domain, and a light chain portion (L1) comprising a first light-chain variable (VL) domain and a CL domain is referred to as H1L1, and a second F(ab) comprising a heavy chain portion (H2) comprising a second heavy-chain variable (VH) domain and a CH1 domain, and a light chain portion (L2) comprising a second light-chain variable (VL) domain and a CL domain is referred to as H2L2. A homodimer comprising only H1L1 in the Fab portions is termed as H1L1/H1L1, and a homodimer comprising only H2L2 in the Fab portions is termed as H2L2/H2L2. A multi-specific antigen-binding molecule comprising H1L1 and H2L2 in the Fab portions is termed as H1L1/H2L2. A third F(ab) comprising a heavy chain portion (H1) comprising the first heavy-chain variable (VH) domain and CH1 domain, and a light chain portion (L2) comprising the second light-chain variable (VL) domain and CL domain is referred to as H1L2, and a fourth F(ab) comprising a heavy chain portion (H2) comprising the second heavy-chain variable (VH) domain and CH1 domain, and a light chain portion (L1) comprising the first light-chain variable (VL) domain and CL domain is referred to as H2L1. A homodimer comprising only H1L2 in the Fab portions is termed as H1L2/H1L2, and a homodimer comprising only H2L1 is termed as H2L1/H2L1. A molecule comprising H1L2 and H2L1 in the Fab portions, namely, a light chain-exchanged molecule of the multi-specific antigen-binding molecule H1L1/H2L2, is referred to as H1L2/H2L1. The amino acid sequences of the homodimers, multi-specific antigen binding molecules, and light chain-exchanged molecules used in the present Example are shown below in Table 1.

**[Table 1]**

| Antibody name | Heavy chain | Light chain | VH | VL | CH | CL |
|---|---|---|---|---|---|---|
| H1L1/H1L1 | Anti-FIX(a) | Anti-FIX(a) | SEQ ID NO: 1 | SEQ ID NO: 2 | SEQ ID NO: 3 | SEQ ID NO: 4 |
| H2L2/H2L2 | Anti-FX | Anti-FX | SEQ ID NO: 5 | SEQ ID NO: 6 | SEQ ID NO: 7 | SEQ ID NO: 8 |
| H1L2/H1L2 | Anti-FIX(a) | Anti-FX | SEQ ID NO: 1 | SEQ ID NO: 6 | SEQ ID NO: 3 | SEQ ID NO: 8 |
| H2L1/H2L1 | Anti-FX | Anti-FIX(a) | SEQ ID NO: 5 | SEQ ID NO: 2 | SEQ ID NO: 7 | SEQ ID NO: 4 |
| H1L1/H1L1 | Anti-EREG | Anti-EREG | SEQ ID NO: 9 | SEQ ID NO: 10 | SEQ ID NO: 11 | SEQ ID NO: 12 |
| H2L2/H2L2 | Anti-GPC3 | Anti-GPC3 | SEQ ID NO: 13 | SEQ ID NO: 14 | SEQ ID NO: 15 | SEQ ID NO: 16 |
| H1L2/H1L2 | Anti-EREG | Anti-GPC3 | SEQ ID NO: 9 | SEQ ID NO: 14 | SEQ ID NO: 11 | SEQ ID NO: 16 |
| H2L1/H2L1 | Anti-GPC3 | Anti-EREG | SEQ ID NO: 13 | SEQ ID NO: 10 | SEQ ID NO: 15 | SEQ ID NO: 12 |
| H1L1/H1L1 | Anti-IL-6R | Anti-IL-6R | SEQ ID NO: 17 | SEQ ID NO: 18 | SEQ ID NO: 11 | SEQ ID NO: 19 |
| H2L2/H2L2 | Anti-KLH | Anti-KLH | SEQ ID NO: 20 | SEQ ID NO: 21 | SEQ ID NO: 15 | SEQ ID NO: 19 |
| H1L2/H1L2 | Anti-IL-6R | Anti-KLH | SEQ ID NO: 17 | SEQ ID NO: 21 | SEQ ID NO: 11 | SEQ ID NO: 19 |
| H2L1/H2L1 | Anti-KLH | Anti-IL-6R | SEQ ID NO: 20 | SEQ ID NO: 18 | SEQ ID NO: 15 | SEQ ID NO: 19 |
| H1L1/H2L2 | Anti-IL-6R | Anti-IL-6R | SEQ ID NO: 17 | SEQ ID NO: 18 | SEQ ID NO: 11 | SEQ ID NO: 19 |
| | Anti-KLH | Anti-KLH | SEQ ID NO: 20 | SEQ ID NO: 21 | SEQ ID NO: 15 | SEQ ID NO: 19 |
| H1L2/H2L1 | Anti-IL-6R | Anti-KLH | SEQ ID NO: 17 | SEQ ID NO: 21 | SEQ ID NO: 11 | SEQ ID NO: 19 |
| | Anti-KLH | Anti-IL-6R | SEQ ID NO: 20 | SEQ ID NO: 18 | SEQ ID NO: 15 | SEQ ID NO: 19 |

### Example 1: Separation of F(ab) fragments contained in anti-FIX(a)/anti-FX bispecific antibody and impurity molecule (light chain-exchanged molecule) thereof

An expression vector encoding the respective homodimers consisting of the heavy and light chains of the anti-FIX(a) and anti-FX antibodies shown in Table 1 was introduced into HEK cells, and the culture supernatant was recovered after culturing the cells for 4 days. Purification by a Protein A column was performed to prepare a sample containing homodimers H1L1/H1L1, H2L2/H2L2, H1L2/H1L2, and H2L1/H2L1. Subsequently, FabRICATOR (Genovis) was added to the sample for enzyme treatment. To the sample, TCEP (Thermo Fisher Scientific) was further added at a final concentration of 5 mM, thereby preparing a sample containing the F(ab) fragments of H1L1, H2L2, H1L2, and H2L1.

CEX analysis was carried out on this sample according to the method described in Example 5. The results are shown in Fig. 1. The peaks derived from the respective F(ab) fragments were observed at different elution times, *i.e.,* F(ab) domains H1L1, H2L2, H1L2, and H2L1 contained in the anti-FIX(a)/anti-FX bispecific antibody and light chain-exchanged molecule were eluted at different retention times.

### Example 2: Separation of F(ab) fragments contained in anti-EREG/anti-GPC3 bispecific antibody and impurity molecule (light chain-exchanged molecule) thereof

An expression vector encoding the respective homologous antibodies consisting of the heavy and light chains of the anti-EREG and anti-GPC3 antibodies shown in Table 1 was introduced into HEK cells, and the culture supernatant was recovered after culturing the cells for 4 days. Purification by a Protein A column was performed to prepare a sample containing homodimers H1L1/H1L1, H2L2/H2L2, H1L2/H1L2, and H2L1/H2L1. Subsequently, GingisKHAN (Genovis) was added to the sample, thereby preparing a sample containing the F(ab) fragments of H1L1, H2L2, H1L2, and H2L1.

CEX analysis was carried out on this sample according to the method described in Example 5. The results are shown in Fig. 2. The peaks derived from the respective F(ab) fragments were observed at different elution times, *i.e*., F(ab) domains H1L1, H2L2, H1L2, and H2L1 contained in the anti-EREG/anti-GPC3 bispecific antibody and light chain-exchanged molecule were eluted at different retention times.

### Example 3: Separation of F(ab) fragments contained in anti-IL-6R/anti-KLH bispecific antibody and impurity molecule (light chain-exchanged molecule) thereof

An expression vector encoding the respective homologous antibodies consisting of the heavy and light chains of the anti-IL-6R and anti-KLH antibodies shown in Table 1 was introduced into HEK cells, and the culture supernatant was recovered after culturing the cells for 4 days. Purification by a Protein A column was performed to prepare a sample containing homodimers H1L1/H1L1, H2L2/H2L2, H1L2/H1L2, and H2L1/H2L1. Subsequently, GingisKHAN (Genovis) was added to the sample, thereby preparing a sample containing the F(ab) fragments of H1L1, H2L2, H1L2, and H2L1.

CEX analysis was carried out on this sample according to the method described in Example 5. The results are shown in Fig. 3. The peaks derived from the respective F(ab) fragments were observed at different elution times, *i.e.,* F(ab) domains H1L1, H2L2, H1L2, and H2L1contained in the anti-IL-6R/anti-KLH bispecific antibody and light chain-exchanged molecule were eluted at different retention times.

### Example 4: Quantification of the content ratio of anti-IL-6R/anti-KLH bispecific antibody and impurity molecule (light chain-exchanged molecule) thereof in mixed sample

Expression vectors encoding the anti-IL-6R/anti-KLH bispecific antibody (H1L1/H2L2) and its light chain-exchanged molecule (H1L2/H2L1) shown in Table 1 were introduced respectively into HEK cells, and the culture supernatants were recovered after culturing the cells for 4 days. Purification by a Protein A column was performed to prepare anti-IL-6R/anti-KLH bispecific antibody H1L1/H2L2 and light chain-exchanged molecule H1L2/H2L1. They were mixed so that the mass ratio of the bispecific antibody to the light chain-exchanged molecule is 90:10, thereby preparing a sample. To this sample, GingisKHAN (Genovis) was added to prepare a sample containing the F(ab) fragments of H1L1, H2L2, H1L2, and H2L1, which were disassembled from the bispecific antibody and the light chain-exchanged molecule.

CEX analysis was carried out on this sample according to the method described in Example 5. The results are shown in Fig. 4. The peaks derived from the respective F(ab) fragments of H1L1, H2L2, H1L2, and H2L1 were observed. The content ratio of the impurity molecule was calculated to be 10%, based on the following calculating formula:
peak area of H1L2 and H2L1 derived from the impurity molecule divided by the total peak area, *i.e.,* (peak area of H1L2 + peak area of H2L1) / (peak area of H1L1 + peak area of H2L2 + peak area of H1L2 + peak area of H2L1).

### Example 5: CEX analysis of sample containing F(ab)

The analysis was carried out using the Prominence (Shimazu) apparatus. For mobile phase A, 20 mM MES-NaOH, pH 5.5 was used, and for mobile phase B, 20 mM MES-NaOH, 500 mM NaCl, pH 5.5 was used. A ProPAC WCX-10 4 x 250 mm (Thermo Fisher Scientific) column was attached and equilibration was carried out at a condition of 0.5 mL/min flow rate, B 1%, and 25°C column temperature. Ten µg of the sample containing the respective F(ab)s prepared was injected into the column. Cation-exchange chromatography (CEX) was performed by increasing B% at a rate of 1%/min while sending the solution at a flow rate of 0.5 mL/min. Peak detection was carried out using absorbance at 280 nm.

### [Industrial Applicability]

The method of the present disclosure allows accurate measurement of the content or content ratio of impurity molecules (*e.g*., light chain-exchanged molecules) in a composition comprising a multi-specific antigen-binding molecule. The method is useful compared to conventional analysis methods which can only obtain estimates using theoretical calculations.

## Claims

1. A method for analyzing a light chain-exchanged molecule in a composition comprising a multi-specific antigen-binding molecule,
wherein the multi-specific antigen-binding molecule is a molecule (H1L1/H2L2) comprising a first F(ab) (H1L1) comprising a first heavy-chain variable domain and a first light-chain variable domain which are directed against a first antigen, and a second F(ab) (H2L2) comprising a second heavy-chain variable domain and a second light-chain variable domain which are directed against a second antigen;
wherein the light chain-exchanged molecule is a molecule (H1L2/H2L1) comprising a third F(ab) (H1L2) comprising the first heavy-chain variable domain and the second light-chain variable domain, and a fourth F(ab) (H2L1) comprising the second heavy-chain variable domain and the first light-chain variable domain;
wherein the analysis method comprises the following steps 1 to 2:
1) treating the composition comprising the multi-specific antigen-binding molecule and thereby generating two or more types of F(ab) fragments; and
2) measuring the two or more types of F(ab) fragments by a separation method based on electric charge or hydrophobic interaction and determining the content of the light chain-exchanged molecule in the composition, or the content ratio of the light chain-exchanged molecule to the multi-specific antigen-binding molecule in the composition.

2. The method of claim 1, wherein step 1) comprises the following steps 1-1 and 1-2:
1-1) cleaving the molecules comprised in the composition and thereby generating a F(ab)'2 fragment; and
1-2) cleaving the F(ab)'2 fragment and thereby generating two or more types of F(ab) fragments.

3. The method of claim 2, wherein step 1-1 is a step of cleaving with protease the Fc side of the hinge region of the molecules comprised in the composition.

4. The method of claim 3, wherein the protease is any one of a bacteria-derived antibody-degrading enzyme, pepsin, and ficin, or a combination thereof.

5. The method of any one of claims 2 to 4, wherein step 1-2 is a step of cleaving the disulfide bonds in the F(ab)'2 fragment with a reducing agent.

6. The method of claim 5, wherein the reducing agent is any one of TCEP, 2-MEA, cysteine, dithiothreitol, 2-mercaptoethanol, 3-mercapto-1,2-propanediol, and TBP, or a combination thereof.

7. The method of claim 1, wherein step 1 is a step of cleaving with protease the F(ab) side of the hinge region of the molecules comprised in the composition.

8. The method of claim 7, wherein the protease is any one of a bacteria-derived antibody-degrading enzyme, papain, and Lys-C, or a combination thereof.

9. The method of any one of claims 1 to 8, wherein step 2 is a step of measuring the two or more types of F(ab) fragments by any one of cation-exchange chromatography, anion-exchange chromatography, hydrophobic interaction chromatography, and reverse-phase chromatography, or a combination thereof.

10. The method of any one of claims 1 to 9, wherein the two or more types of F(ab) fragments generated in step 1 are different in isoelectric point from each other.

11. The method of any one of claims 1 to 10, wherein the multi-specific antigen-binding molecule (H1L1/H2L2) is a molecule in which an amino acid residue has been modified so as to provide an isoelectric point different from those of the following impurities (1) to (8):
(1) a homodimer (H1L1/H1L1) of the first F(ab) (H1L1), which comprises the first heavy-chain variable domain and the first light-chain variable domain;
(2) a homodimer (H2L2/H2L2) of the second F(ab) (H2L2), which comprises the second heavy-chain variable domain and the second light-chain variable domain;
(3) a homodimer (H1L2/H1L2) of the third F(ab) (H1L2), which comprises the first heavy-chain variable domain and the second light-chain variable domain;
(4) a homodimer (H2L1/H2L1) of the fourth F(ab) (H2L1), which comprises the second heavy-chain variable domain and the first light-chain variable domain;
(5) a heterodimer (H1L1/H1L2) comprising the first F(ab) (H1L1), which comprises the first heavy-chain variable domain and the first light-chain variable domain, and the third F(ab) (H1L2), which comprises the first heavy-chain variable domain and the second light-chain variable domain;
(6) a heterodimer (H1L1/H2L1) comprising the first F(ab) (H1L1), which comprises the first heavy-chain variable domain and the first light-chain variable domain, and the fourth F(ab) (H2L1), which comprises the second heavy-chain variable domain and the first light-chain variable domain;
(7) a heterodimer (H2L2/H1L2) comprising the second F(ab) (H2L2), which comprises the second heavy-chain variable domain and the second light-chain variable domain, and the third F(ab) (H1L2), which comprises the first heavy-chain variable domain and the second light-chain variable domain; and
(8) a heterodimer (H2L2/H2L1) comprising the second F(ab) (H2L2), which comprises the second heavy-chain variable domain and the second light-chain variable domain, and the fourth F(ab) (H2L1), which comprises the second heavy-chain variable domain and the first light-chain variable domain.

12. The method of claim 11, wherein the composition comprising the multi-specific antigen-binding molecule has had impurities (1) to (8) removed through a purification step that uses the difference in isoelectric point.

13. The method of any one of claims 1 to 12, wherein the multi-specific antigen-binding molecule is a bispecific antibody.

14. The method of claim 13, wherein the bispecific antibody is an antibody in which at least one amino acid residue selected from the amino acid residues at positions 1, 3, 5, 8, 10, 12, 13, 15, 16, 19, 23, 25, 26, 39, 42, 43, 44, 46, 68, 71, 72, 73, 75, 76, 81, 82b, 83, 85, 86, 97, 105, 108, 110, and 112 according to Kabat numbering in the heavy-chain variable domain, and the amino acid residues at positions 137, 196, 203, 214, 217, 233, 268, 274, 276, 297, 355, 392, 419, and 435 according to EU numbering in the heavy-chain constant domain has been modified.

15. The method of any one of claims 1 to 14, wherein the composition comprising a multi-specific antigen-binding molecule or a bispecific antibody is a pharmaceutical composition.
